# EUROPEAN PATENT SPECIFICATION

(11) **EP 1 713 810 B1**
(45) Date of publication and mention of the grant of the patent: **29.12.2010**
(21) Application number: 05716606.8
(22) Date of filing: 01.02.2005
(51) Int. Cl.: C07D 487/08, C07D 471/08, A61K 31/4995, A61K 31/551, A61P 25/00

(54) **DIAZABICYCLIC ARYL DERIVATIVES AS NICOTINIC ACETYLCHOLINE RECEPTOR LIGANDS**
DIAZABICYCLISCHE ARYLDERIVATE ALS LIGANDEN DES NIKOTINISCHEN ACETYLCHOLINREZEPTORS
DERIVES D'ARYLE DIAZABICYCLIQUE CONSTITUANT DES LIGANDS DU RECEPTEUR DE L'ACETYLCHOLINE NICOTINIQUE

(30) Priority: 04.02.2004 DK 200400169; 05.02.2004 US 541754 P; 28.05.2004 US 574946 P; 28.05.2004 DK 200400839
(43) Date of publication of application: 25.10.2006
(73) Proprietor: NeuroSearch A/S, 2750 Ballerup (DK)
(72) Inventor: PETERS, Dan, DK-2750 Ballerup (DK); OLSEN, Gunnar M., DK-2750 Ballerup (DK); NIELSEN, Elsebet Østergaard, DK-2750 Ballerup (DK); JØRGENSEN, Tino Dyhring, DK-2750 Ballerup (DK); AHRING, Philip K., DK-2750 Ballerup (DK); TIMMERMANN, Daniel B., DK-2750 Ballerup (DK)
(86) International application number: PCT/EP2005/050405
(87) International publication number: WO 2005/075482

(56) References cited:
- WO-A-00/58311
- WO-A-2004/016617
- WO-A1-2004/076453
- DE COSTA B R ET AL: "Synthesis and evaluation of comformationally restricted N-[2-(3,4-dichlorophenyl)ethyl]-N-methyl-2 -(1-pyrrolidinyl)ethylamines at sigma receptors. 2. Piperazines, bicyclic amines, bridged bicyclic amines, and miscellaneous compounds" JOURNAL OF MEDICINAL CHEMISTRY, AMERICAN CHEMICAL SOCIETY. WASHINGTON, US, vol. 36, no. 16, 1993, pages 2311-2320, XP002125229 ISSN: 0022-2623 cited in the application

## Description

### TECHNICAL FIELD

This invention relates to novel diazabicyclic aryl derivatives, which are found to be cholinergic ligands at the nicotinic acetylcholine receptors and modulators of the monoamine receptors and transporters. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exert its biological effect via two types of cholinergic receptors, the muscarinic Acetyl Choline Receptors (mAChR) and the nicotinic Acetyl Choline Receptors (nAChR).

As it is well established that muscarinic acetylcholine receptors dominate quantitatively over nicotinic acetylcholine receptors in the brain area important to memory and cognition, and much research aimed at the development of agents for the treatment of memory related disorders have focused on the synthesis of muscarinic acetylcholine receptor modulators.

Recently, however, an interest in the development of nAChR modulators has emerged. Several diseases are associated with degeneration of the cholinergic system i.e. senile dementia of the Alzheimer type, vascular dementia and cognitive impairment due to the organic brain damage disease related directly to alcoholism.

WO 00/58311 discloses 1,4-diazabicyclo[3.2.2]nonane-4-carboxylates and carboxamide derivatives useful as inhibitors of the nicotinic α7 receptor subtype. Other 1,4-diazabicyclo[3.2.2]nonane-4-methanone derivatives are not disclosed.

### SUMMARY OF THE INVENTION

The present invention is devoted to the provision novel modulators of the nicotinic and/or of the monoamine receptors, which modulators are useful for the treatment of diseases or disorders related to the cholinergic receptors, and in particular the nicotinic acetylcholine receptor (nAChR), the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides diazabicyclic aryl derivatives of Formula IV 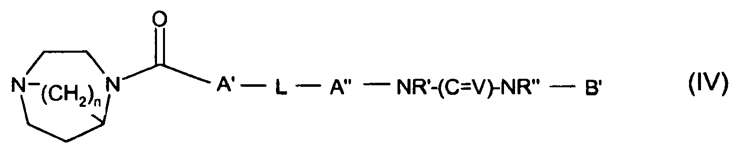 any of its enantiomers or any mixture of its enantiomers, or a pharmaceutically acceptable addition salt thereof, wherein
n is 2;
L represents a single (covalent) bond;
A' represents a furanyl group;
A" represents a phenyl group;
R' represents hydrogen, C₁₋₆-alkyl or -(C=O)-NH-B'-;
R" represents hydrogen, C₁₋₆-alkyl, phenyl or benzyl;
V represents O, S or NH; and
B' represents C₁₋₆-alkyl, phenyl, benzyl or pyridyl; which phenyl, benzyl and pyridyl groups are optionally substituted one or two times with substituents selected from the group consisting of hydroxy, C₁₋₆-alkoxy, halo, trifluoromethyl, nitro, amino, C₁₋₆-alkyl-carbonyl-amino, *N*-C₁₋₆-alkyl-amino-carbonyl-amino (N-C₁₋₆-alkyl-ureido), *N,N-*di-C₁₋₆-alkyl-amino-carbonyl-amino (N,N-di-C₁₋₆-alkyl-ureido) and sulfamoyl.

In a second aspect the invention provides pharmaceutical compositions comprising a therapeutically effective amount of the diazabicyclic aryl derivative of the invention, or a pharmaceutically acceptable addition salt thereof, together with at least one pharmaceutically acceptable carrier or diluent.

Viewed from another aspect the invention relates to the use of the diazabicyclic aryl derivative of the invention, or a pharmaceutically acceptable addition salt thereof, for the manufacture of pharmaceutical compositions/medicaments for the treatment, prevention or alleviation of a disease or a disorder or a condition of a mammal, including a human, which disease, disorder or condition is responsive to modulation of cholinergic receptors.

Other objects of the invention will be apparent to the person skilled in the art from the following detailed description and examples.

### DETAILED DISCLOSURE OF THE INVENTION

### Diazabicyclic Aryl Derivatives

In its first aspect the invention provides diazabicyclic aryl derivatives of Formula IV 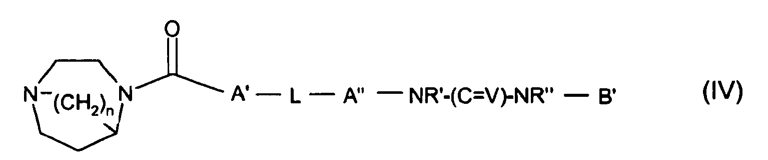 any of its enantiomers or any mixture of its enantiomers, or a pharmaceutically acceptable addition salt thereof, wherein
n is 2;
L represents a single (covalent) bond;
A' represents a furanyl group;
A" represents a phenyl group;
R' represents hydrogen, C₁₋₆-alkyl or -(C=O)-NH-B'-;
R" represents hydrogen, C₁₋₆-alkyl, phenyl or benzyl;
V represents O, S or NH; and
B' represents C₁₋₆-alkyl, phenyl, benzyl or pyridyl; which phenyl, benzyl and pyridyl groups are optionally substituted one or two times with substituents selected from the group consisting of hydroxy, C₁₋₆-alkoxy, halo, trifluoromethyl, nitro, amino, C₁₋₆-alkyl-carbonyl-amino, *N*-C₁₋₆-alkyl-amino-carbonyl-amino (N-C₁₋₆-alkyl-ureido), *N,N-*di-C₁₋₆-alkyl-amino-carbonyl-amino (N,N-di-C₁₋₆-alkyl-ureido) and sulfamoyl.

In a most preferred embodiment the diazabicyclic aryl derivative of the invention is
1-{4-[5-(1,4-Diaza-bicyclo[3.2.2]nonane-4-carbonyl)-furan-2-yl]-phenyl}-3-ethyl-urea;
1-{4-[5-(1,4-Diaza-bicyclo[3.2.2]nonane-4-carbonyl)-furan-2-yl]-phenyl}-3-phenyl-urea;
1-{4-[5-(1,4-Diaza-bicyclo[3.2.2]nonane-4-carbonyl)-furan-2-yl]-phenyl}-3-(2-nitrophenyl)-urea;
1-{4-[5-(1,4-Diaza-bicyclo[3.2.2]nonane-4-carbonyl)-furan-2-yl]-phenyl}-3-(2-acetylaminophenyl)-urea;
1-{4-[5-(1,4-Diaza-bicyclo[3.2.2]nonane-4-carbonyl)-furan-2-yl]-phenyl}-3-(2-aminophenyl)-urea;
1-{4-[5-(1,4-Diaza-bicyclo[3.2.2]nonane-4-carbonyl)-furan-2-yl]-phenyl}-3-(5-chloro-2-methoxyphenyl)-thiourea;
1-{4-[5-(1,4-Diaza-bicyclo[3.2.2]nonane-4-carbonyl)-furan-2-yl]-phenyl}-3-(5-chloro-2-methoxy-phenyl)-urea;
1-{4-[5-(1,4-Diaza-bicyclo[3.2.2]nonane-4-carbonyl)-furan-2-yl]-phenyl}-3-benzyl-urea;
1-{4-[5-(1,4-Diaza-bicyclo[3.2.2]nonane-4-carbonyl)-furan-2-yl]-phenyl}-1'-benzylaminocarbonyl-3-benzyl-urea;
1-{4-[5-(1,4-Diaza-bicyclo[3.2.2]nonane-4-carbonyl)-furan-2-yl]-phenyl}-3-(2-chlorophenyl)-urea;
1-{3-[5-(1,4-Diaza-bicyclo[3.2.2]nonane-4-carbonyl)-furan-2-yl]-phenyl}-3-phenyl-urea;
1-{4-[5-(1,4-Diaza-bicyclo[3.2.2]nonane-4-carbonyl)-furan-2-yl]-phenyl}-3-(2-fluorophenyl)-urea;
1-{4-[5-(1,4-Diaza-bicyclo[3.2.2]nonane-4-carbonyl)-furan-2-yl]-phenyl}-3-(3-fluorophenyl)-urea;
1-{4-[5-(1,4-Diaza-bicyclo[3.2.2]nonane-4-carbonyl)-furan-2-yl]-phenyl}-3-(2-trifluoromethylphenyl)-urea;
1-[2-(3-{4-[5-(1,4-Diaza-bicyclo[3.2.2]nonane-4-carbonyl)-furan-2-yl]-phenyl}-ureido)-phenyl]-3-ethyl-urea;
1-{4-[5-(1,4-Diaza-bicyclo[3.2.2]nonane-4-carbonyl)-furan-2-yl]-phenyl}-3-(3-trifluoromethylphenyl)-urea; or
1-{3-[5-(1,4-Diaza-bicyclo[3.2.2]nonane-4-carbonyl)-furan-2-yl]-phenyl}-3-ethyl-urea;
or an enantiomer or a mixture of its enantiomers, or a pharmaceutically acceptable addition salt thereof.

### Definition of Substituents

In the context of this invention an alkyl group designates a univalent saturated, straight or branched hydrocarbon chain. The hydrocarbon chain preferably contain of from one to eighteen carbon atoms (C₁₋₁₈-alkyl), more preferred of from one to six carbon atoms (C₁₋₆-alkyl; lower alkyl), including pentyl, isopentyl, neopentyl, tertiary pentyl, hexyl and isohexyl. In a preferred embodiment alkyl represents a C₁₋₄-alkyl group, including butyl, isobutyl, secondary butyl, and tertiary butyl. In another preferred embodiment of this invention alkyl represents a C₁₋₃-alkyl group, which may in particular be methyl, ethyl, propyl or isopropyl.

In the context of this invention an alkoxy group designates an "alkyl-O-" group, wherein alkyl is as defined above. Examples of preferred alkoxy groups of the invention include methoxy and ethoxy.

### Pharmaceutically Acceptable Salts

The diazabicyclic aryl derivative of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds may also be contemplated as pharmaceutically acceptable salts. Preferred "onium salts" include the alkyl-onium salts, the cycloalkyl-onium salts, and the cycloalkylalkylonium salts. Particularly preferred onium salts of the invention include those created at the N' position according to the following Formula I'

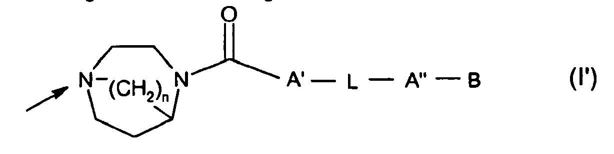

### Steric Isomers

The chemical compounds of the present invention may exist in (+) and (-) forms as well as in racemic forms. The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or I- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials.

### Methods of Producing Diazabicyclic Aryl Derivatives

The diazabicyclic aryl derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention is devoted to the provision novel ligands and modulators of the nicotinic receptors, which ligands and modulators are useful for the treatment of diseases or disorders related to the cholinergic receptors, and in particular the nicotinic acetylcholine receptor (nAChR). Preferred compounds of the invention show a pronounced nicotinic acetylcholine α7 receptor subtype selectivity.

The compounds of the present invention may in particular be agonists, partial agonists, antagonists and/or allosteric modulators of the nicotinic acetylcholine receptor.

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In a preferred embodiment the compounds of the invention are used for the treatment of diseases, disorders, or conditions relating to the central nervous system. Such diseases or disorders includes anxiety, cognitive disorders, learning deficit, memory deficits and dysfunction, Alzheimer's disease, attention deficit, attention deficit hyperactivity disorder (ADHD), Parkinson's disease, Huntington's disease, Amyotrophic Lateral Sclerosis, Gilles de la Tourette's syndrome, psychosis, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

In a preferred embodiment diseases, disorders, or conditions relating to the central nervous system for which the compounds of the invention are used are cognitive disorders, psychosis, schizophrenia and/or depression.

In another preferred embodiment the compounds of the invention may be useful for the treatment of diseases, disorders, or conditions associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

In yet another preferred embodiment the compounds of the invention may be useful for the treatment of endocrine disorders, such as thyrotoxicosis, pheochromocytoma, hypertension and arrhythmias.

In still another preferred embodiment the compounds of the invention may be useful for the treatment of neurodegenerative disorders, including transient anoxia and induced neuro-degeneration.

In even another preferred embodiment the compounds of the invention may be useful for the treatment of inflammatory diseases, disorders, or conditions, including inflammatory skin disorders such as acne and rosacea, Crohn's disease, inflammatory bowel disease, ulcerative colitis, and diarrhoea.

In still another preferred embodiment the compounds of the invention may be useful for the treatment of mild, moderate or even severe pain of acute, chronic or recurrent character, as well as pain caused by migraine, postoperative pain, and phantom limb pain. The pain may in particular be neuropathic pain, chronic headache, central pain, pain related to diabetic neuropathy, to post therapeutic neuralgia, or to peripheral nerve injury.

Finally the compounds of the invention may be useful for the treatment of withdrawal symptoms caused by termination of use of addictive substances. Such addictive substances include nicotine containing products such as tobacco, opioids such as heroin, cocaine and morphine, benzodiazepines and benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

### Pharmaceutical Compositions

In another aspect the invention provides novel pharmaceutical compositions comprising a therapeutically effective amount of diazabicyclic aryl derivative of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the diazabicyclic aryl derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by the skilled person by use of standard methods and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depend on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### EXAMPLES

The invention is further illustrated with reference to the following examples, which are not intended to be in any way limiting to the scope of the invention as claimed.

### Example 1

### Preparatory Example

All reactions involving air sensitive reagents or intermediates were performed under nitrogen and in anhydrous solvents.

### 1,4-Diazabicyclo[3.2.2]nonan-3-one (Intermediate compound)

32.33 g (200 mmol) of 3-Quinuclidinone hydrochloride was dissolved in 75 ml of water, and to the solution of hydroxylamine hydrochloride (16.4 g; 236 mmol) and CH₃CO₂Na · 3H₂O (80 g; 588 mmol) was added. The mixture was stirred at 70°C for 1 hour. Then NaCl (10 g) was dissolved in the mixture and was cooled to 0°C. Separated crystals were filtered and carefully dried. The thus obtained crude 3-quinuclidone oxime (approx. 30 g) was used in the next step of the synthesis without further purification.

Polyphosphoric acid (180 g) of was heated to 100°C and crude 3-quinuclidone oxime (30 g) was added portion-wise. The reaction mixture was heated at 130°C for 20 minutes. The mixture was cooled to room temperature, and 50 ml of water was added. The mass was carefully homogenised, the mixture was poured into of ice (100 g). The mixture was made alkaline (pH 12) by adding sodium hydroxide. The mixture was extracted with chloroform (2 x 400 ml). The extract was dried over sodium sulphate and the solvent was removed under reduced pressure.

Yield of the mixture of the products 1,4-diazabicyclo[3.2.2]nonan-3-one and 1,3-diazabicyclo[3.2.2]nonan-4-one was 19.02 g (68%). The mixture of isomers was crystallized from 80 ml of dioxane to yield 1,4-diazabicyclo[3.2.2]nonan-3-one (5.12 g; 18%). The solvent from filtrate was distilled off, flash chromatography (with acetone) of the residue gave of 1,3-diazabicyclo[3.2.2]nonan-4-one (8.91 g; 32%).

### 1,4-Diazabicyclo[3.2.2]nonane [J. Med. Chem. 1993 36 2311-2320]

### (Intermediate compound)

1,4-Diazabicyclo[3.2.2]nonan-3-one (5.12 g; 36 mmol) was dissolved in tetrahydrofuran (50 ml), litium aluminium hydride 2.28 g (60 mmol) was added to the solution and the reaction mixture was refluxed for 36 hours. After cooling the reaction mixture to room temperature, water (2.3 ml) was added dropwise and the mixture was filtered. The solvent was removed from the filtrate by rotavapor at reduced pressure. The formed substance was distilled with Kugelrohr (0.5 mBar; 70°C). Yield of the title compound 3.11 g (68%).

### Method A

### 1-{4-[5-(1,4-Diaza-bicyclo[3.2.2]nonane-4-carbonyl)-furan-2-yl]-phenyl}-3-(2-aminophenyl)-urea free base (Compound A1)

A mixture of 1-{4-[5-(1,4-diaza-bicyclo[3.2.2]nonane-4-carbonyl)-furan-2-yl]-phenyl}-3-(2-nitrophenyl)-urea (0.63 g; 1.32 mmol), palladium on carbon (0.60 g; 5%) and ethanol (50 ml) was stirred under hydrogen. The crude mixture was filtered and purified by silica gel chromatography, using a mixture of dichloromethane : methanol (9:1) and 1% methanol as eluent. Yield 0.50 g (85%). Mp. 174°C.

### (1,4-Diaza-bicyclo[3.2.2]non-4-yl)-5-(4-aminophenyl)-furan-2-yl-methanone fumaric acid salt (Intermediate Compound)

The title compound was prepared according to method A from (1,4-diaza-bicyclo[3.2.2]non-4-yl)-5-(4-nitrophenyl)-furan-2-yl-methanone. Mp. 227.8°C.

### (1,4-Diaza-bicyclo[3.2.2]non-4-yl)-5-(4-nitrophenyl)-furan-2-yl-methanone hydrochloric acid salt (Intermediate Compound)

A mixture of 5-(4-nitrophenyl)-2-furoyl chloride (1.0 g; 4,0 mmol), 1,4-diazabicyclo[3.2.2]nonane (0.50 g; 4.0 mmol) and 1,2-dimethoxyethane (20 ml) was stirred for 15 hours at room temperature. The title compound was filtered. Yield 1.4 g (93%). Mp. 298.2°C.

### 5-(4-Nitrophenyl)-2-furoyl chloride (Intermediate Compound)

Was prepared by stirring a mixture of 5-(4-nitrophenyl)-2-furoic acid (1.0 g; 4.3 mmol) and thionyl chloride (10 ml) at reflux for 2 hours. The mixture was evaporated and co-evaporated with anhydrous toluene. The acid chloride was used without further purification.

### Method B

### 1-{4-[5-(1,4-Diaza-bicyclo[3.2.2]nonane-4-carbonyl)-furan-2-yl]-phenyl}-3-(2-acetylaminophenyl)-urea fumaric acid salt (Compound B2)

A mixture of 1-{4-[5-(1,4-Diaza-bicyclo[3.2.2]nonane-4-carbonyl)-furan-2-yl]-phenyl}-3-(2-aminophenyl)-urea (0.21 g; 0.47 mmol), acetic anhydride (144 mg; 1.42 mmol) and dichloromethane (20 ml) was stirred for 4 hours. Aqueous sodium hydroxide (10 ml; 1 M) was added followed by extraction with dichloromethane (3 x 10 ml). The crude mixture was purified by silica gel chromatography, using a mixture of dichloromethane : methanol (9:1) and 1% methanol as eluent. Yield: 174 mg (79%). The corresponding salt was obtained by addition of a diethyl ether and methanol mixture (9:1) saturated with fumaric acid Mp. 159-169°C.

### Method C

### 1-{4-[5-(1,4-Diaza-bicyclo[3.2.2]nonane-4-carbonyl)-furan-2-yl]-phenyl}-3-phenyl-urea free base (Compound C1)

A mixture of (1,4-diaza-bicyclo[3.2.2]non-4-yl)-5-(4-aminophenyl)-furan-2-yl-methanone (0.50 g; 1.6 mmol), phenyl-isocyanate (498 mg; 4.18 mmol) and dichloromethane (50 ml) was stirred for 15 hours. Aqueous sodium hydroxide (10 ml; 1 M) was added followed by extraction with dichloromethane (3 x 10 ml). The crude mixture was purified by silica gel chromatography, using a mixture of dichloromethane : methanol (9:1) and 1% methanol as eluent. Yield 0.16 g (23%). Mp. 153-164°C.

### 1-{4-[5-(1,4-Diaza-bicyclo[3.2.2]nonane-4-carbonyl)-furan-2-yl]-phenyl}-3-(2-nitrophenyl)-urea fumaric acid salt (Compound C2)

The title compound was prepared according to Method C from 2-nitrophenylisocyanate. Mp. 198-202°C.

### 1-{4-[5-(1,4-Diaza-bicyclo[3.2.2]nonane-4-carbonyl)-furan-2-yl]-phenyl}-3-ethyl-urea fumaric acid salt (Compound C3)

The title compound was prepared according to Method C from ethylisocyanate. Mp. 167-171 °C.

### 1-{4-[5-(1,4-Diaza-bicyclo[3.2.2]nonane-4-carbonyl)-furan-2-yl]-phenyl}-3-phenylthiourea free base (Compound C4)

The title compound was prepared according to Method C from phenylisothiocyanate. Mp. 171.4-174.7°C.

### 1-{4-[5-1,4-Diaza-bicyclo[3.2.2]nonane-4-carbonyl)-furan-2-yl}-phenyl}-3-(5-chloro-2-methoxy Phenyl-urea free base (Compound C5)

The title compound was prepared according to Method C from 5-chloro-2-methoxyphenylisocyanate. Isolated as an oil.

### 1-{4-[5-(1,4-Diaza-bicyclo[3.2.2]nonane-4-carbonyl)-furan-2-yl]-phenyl}-3-benzyl-urea free base (Compound C6)

The title compound was prepared according to Method C from benzylisocyanate. Isolated as an oil.

### 1-{4-[5-(1,4-Diaza-bicyclo[3.2.2]nonane-4-carbonyl)-furan-2-yl]-phenyl}-1'-benzylaminocarbonyl-3-benzyl-urea free base (Compound C7)

The title compound was prepared according to Method C from benzylisothiocyanate. Mp. 105-130°C.

### 1-{4-[5-(1,4-Diaza-bicyclo[3.2.2]nonane-4-carbonyl)-furan-2-yl]-phenyl}-3-(2-chlorophenyl)-urea free base (Compound C8)

The title compound was prepared according to Method C from 2-chlorophenylisocyanate. Mp. 200-211°C (decomp.).

### 1-{3-[5-(1,4-Diaza-bicyclo[3.2.2]nonane-4-carbonyl)-furan-2-yl]-phenyl}-3-phenyl-urea free base (Compound C9)

The title compound was prepared according to Method C from (1,4-diaza-bicyclo[3.2.2]non-4-yl)-5-(3-aminophenyl)-furan-2-yl-methanone and phenylisocyanate. Mp. 125-130°C.

### 1-{4-[5-(1,4-Diaza-bicyclo[3.2.2]nonane-4-carbonyl)-furan-2-yl]-phenyl}-3-(2-fluorophenyl)-urea free base (Compound C10)

The title compound was prepared according to Method C from 2-fluorophenylisocyanate. Mp. 241°C (decomp.).

### 1-{4-[5-(1,4-Diaza-bicyclo[3.2.2]nonane-4-carbonyl)-furan-2-yl]-phenyl}-3-(3-fluorophenyl)-urea free base (Compound C11)

The title compound was prepared according to Method C from 3-fluorophenylisocyanate. Mp. 230°C.

### 1-{4-[5-(1,4-Diaza-bicyclo[3.2.2]nonane-4-carbonyl)-furan-2-yl]-phenyl}-3-(2-trifluoromethylphenyl)-urea free base (Compound C12)

The title compound was prepared according to Method C from 2-trifluoromethylphenylisocyanate. Mp. 253°C (decomp.).

### 1-[2-(3-{4-[5-(1,4-Diaza-bicyclo[3.2.2]nonane-4-carbonyl)-furan-2-yl]-phenyl}-ureido)-phenyl]-3-ethyl-urea fumaric acid salt (Compound C13)

The title compound was prepared according to Method C from 1-(2-aminophenyl)-3-{4-[5-(1,4-diaza-bicyclo[3.2.2]nonane-4-carbonyl)-furan-2-yl]-phenyl}-urea and ethylisocyanate. Mp. 162.5-165.5°C.

### 1-{4-[5-(1,4-Diaza-bicyclo[3.2.2]nonane-4-carbonyl)-furan-2-yl]-phenyl}-3-(3-trifluoromethylphenyl)-urea free base (Compound C14)

The title compound was prepared according to Method C from 3-trifluoromethylphenylisocyanate. Mp. 171°C.

### 1-{3-[5-(1,4-Diaza-bicyclo[3.2.2]nonane-4-carbonyl)-furan-2-yl]-phenyl}-3-ethyl-urea fumaric acid salt (Compound C15)

The title compound was prepared according to Method C from ethylisocyanate. Mp. 158-161°C.

### Example 2

### In vitro Inhibition of ³H-α-Bungarotoxine Binding in Rat Brain

In this example the affinity of the compounds of the invention for binding to α₇-subtype of nicotinic receptors is determined.

α-Bungarotoxine is a peptide isolated from the venom of the Elapidae snake *Bungarus multicinctus*. It has high affinity for neuronal and neuromuscular nicotinic receptors, where it acts as a potent antagonist. ³H-α-Bungarotoxine labels nicotinic acetylcholine receptors formed by the α₇ subunit isoform found in brain and the α₁ isoform in the neuromuscular junction.

### Tissue preparation

Preparations are performed at 0-4°C. Cerebral cortices from male Wistar rats (150-250 g) are homogenised for 10 seconds in 15 ml of 20 mM Hepes buffer containing 118 mM NaCl, 4.8 mM KCI, 1.2 mM MgSO₄ and 2.5 mM CaCl₂ (pH 7.5) using an Ultra-Turrax homogeniser. The tissue suspension is subjected to centrifugation at 27,000 x g for 10 minutes. The supernatant is discarded and the pellet is washed twice by centrifugation at 27,000 x g for 10 minutes in 20 ml of fresh buffer, and the final pellet is then re-suspended in fresh buffer containing 0.01 % BSA (35 ml per g of original tissue) and used for binding assays.

### Assay

Aliquots of 500 µl of homogenate are added to 25 µl of test solution and 25 µl of ³H-α-bungarotoxine (2 nM, final concentration) and mixed and incubated for 2 hours at 37°C. Non-specific binding is determined using (-)-nicotine (1 mM, final concentration). After incubation, the samples are added 5 ml of ice-cold Hepes buffer containing 0.05% PEI and poured directly onto Whatman GF/C glass fibre filters (pre-soaked in 0.1 % PEI for at least 6 hours) under suction, and immediately washed with 2 x 5 ml ice-cold buffer.

The amount of radioactivity on the filters is determined by conventional liquid scintillation counting. Specific binding is total binding minus non-specific binding.

The test value is given as an IC₅₀ (the concentration of the test substance which inhibits the specific binding of ³H-α-bungarotoxin by 50%).

The results of these experiments are presented in Table 1 below.

**Table 1**

| Inhibition of ³H-α-Bungarotoxine Binding | |
|---|---|
| **Compound No.** | **IC₅₀ (**µ**M)** |
| A1 | 0.0012 |
| B2 | 0.0016 |
| C3 | 0.00056 |

## Claims

1. A diazabicyclic aryl derivative represented by Formula IV 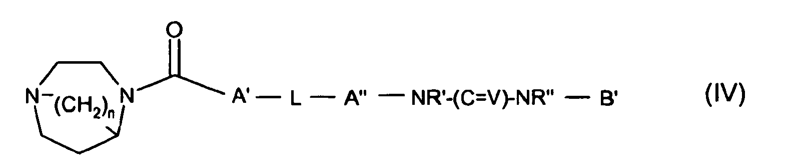 any of its enantiomers or any mixture of its enantiomers, or a pharmaceutically-acceptable addition salt thereof, wherein
n is 2;
L represents a single (covalent) bond;
A' represents a furanyl group;
A" represents a phenyl group;
R' represents hydrogen, C₁₋₆-alkyl or -(C=O)-NH-B'-;
R" represents hydrogen, C₁₋₆-alkyl, phenyl or benzyl;
V represents O, S or NH; and
B' represents C₁₋₆-alkyl, phenyl, benzyl or pyridyl; which phenyl, benzyl and pyridyl groups are optionally substituted one or two times with substituents selected from the group consisting of hydroxy, C₁₋₆-alkoxy, halo, trifluoromethyl, nitro, amino, C₁₋₆-alkyl-carbonyl-amino, *N*-C₁₋₆-alkyl-amino-carbonyl-amino (N-C₁₋₆-alkyl-ureido), *N,N*-di-C₁₋₆-alkyl-amino-carbonyl-amino (N,N-di-C₁₋₆-alkyl-ureido) and sulfamoyl.

2. The diazabicyclic aryl derivative of claim 1, which is
1-{4-[5-(1,4-Diaza-bicyclo[3.2.2]nonane-4-carbonyl)-furan-2-yl]-phenyl}-3-ethyl-urea;
1-{4-[5-(1,4-Diaza-bicyclo[3.2.2]nonane-4-carbonyl)-furan-2-yl]-phenyl}-3-phenyl-urea;
1-{4-[5-(1,4-Diaza-bicyclo[3.2.2]nonane-4-carbonyl)-furan-2-yl]-phenyl}-3-(2-nitrophenyl)-urea;
1-{4-[5-(1,4-Diaza-bicyclo[3.2.2]nonane-4-carbonyl)-furan-2-yl]-phenyl}-3-(2-acetylaminophenyl)-urea;
1-{4-[5-(1,4-Diaza-bicyclo[3.2.2]nonane-4-carbonyl)-furan-2-yl]-phenyl}-3-(2-aminophenyl)-urea;
1-{4-[5-(1,4-Diaza-bicyclo[3.2.2]nonane-4-carbonyl)-furan-2-yl]-phenyl}-3-(5-chloro-2-methoxyphenyl)-thiourea;
1-{4-[5-(1,4-Diaza-bicyclo[3.2.2]nonane-4-carbonyl)-furan-2-yl]-phenyl}-3-(5-chloro-2-methoxy-phenyl)-urea;
1-{4-[5-(1,4-Diaza-bicyclo[3.2.2]nonane-4-carbonyl)-furan-2-yl]-phenyl}-3-benzyl-urea;
1-{4-[5-(1,4-Diaza-bicyclo[3.2.2]nonane-4-carbonyl)-furan-2-yl]-phenyl}-1'-benzylaminocarbonyl-3-benzyl-urea;
1-{4-[5-(1,4-Diaza-bicyclo[3.2.2]nonane-4-carbonyl)-furan-2-yl]-phenyl}-1'-benzylaminocarbonyl-3-benzyl-urea;
1-{4-[5-(1,4-Diaza-bicyclo[3.2.2]nonane-4-carbonyl)-furan-2-yl]-phenyl}-3-(2-chlorophenyl)-urea;
1-{3-[5-(1,4-Diaza-bicyclo[3.2.2]nonane-4-carbonyl)-furan-2-yl]-phenyl}-3-phenyl-urea;
1-{4-[5-(1,4-Diaza-bicyclo[3.2.2]nonane-4-carbonyl)-furan-2-yl]-phenyl}-3-(2-fluorophenyl)-urea;
1-{4-[5-(1,4-Diaza-bicyclo[3.2.2]nonane-4-carbonyl)-furan-2-yl]-phenyl}-3-(3-fluorophenyl)-urea;
1-{4-[5-(1,4-Diaza-bicyclo[3.2.2]nonane-4-carbonyl)-furan-2-yl]-phenyl}-3-(2-trifluoromethylphenyl)-urea;
1-[2-(3-{4-[5-(1,4-Diaza-bicyclo[3.2.2]nonane-4-carbonyl)-furan-2-yl]-phenyl}-ureido)-phenyl]-3-ethyl-urea;
1-{4-[5-(1,4-Diaza-bicyclo[3.2.2]nonane-4-carbonyl)-furan-2-yl]-phenyl}-3-(3-trifluoromethylphenyl)-urea; or
1-{3-[5-(1,4-Diaza-bicyclo[3.2.2]nonane-4-carbonyl)-furan-2-yl]-phenyl}-3-ethyl-urea;
or an enantiomer or a mixture of its enantiomers, or a pharmaceutically-acceptable addition salt thereof.

3. A pharmaceutical composition comprising a therapeutically effective amount of a diazabicyclic aryl derivative of either one of claims 1-2, or an enantiomer or a mixture of its enantiomers, or a pharmaceutically acceptable addition salt thereof, together with at least one pharmaceutically acceptable carrier or diluent.

4. Use of a diazabicyclic aryl derivative of either one of claims 1-2, or an enantiomer or a mixture of its enantiomers, or a pharmaceutically acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors.

5. The use according to claim 4, wherein the disease, disorder or condition is anxiety, cognitive disorders, learning deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania and jet-lag.

6. The use according to claim 4, wherein the disease, disorder or condition are associated with smooth muscle contractions, including convulsive disorders, angina pectoris, premature labour, convulsions, diarrhoea, asthma, epilepsy, tardive dyskinesia, hyperkinesia, premature ejaculation and erectile difficulty.

7. The use according to claim 4, wherein the disease, disorder or condition is related to the endocrine system, such as thyrotoxicosis, pheochromocytoma, hypertension and arrhythmias.

8. The use according to claim 4, wherein the disease, disorder or condition is a neurodegenerative disorders, including transient anoxia and induced neuro-degeneration.

9. The use according to claim 4, wherein the disease, disorder or condition is an inflammatory disorder, including inflammatory skin disorders such as acne and rosacea, Crohn's disease, inflammatory bowel disease, ulcerative colitis and diarrhoea.

10. The use according to claim 4, wherein the disease, disorder or condition is mild, moderate or even severe pain of acute, chronic or recurrent character, as well as neuropathic pain and pain caused by migraine, postoperative pain, phantom limb pain, neuropathic pain, chronic headache, central pain, pain related to diabetic neuropathy, to post therapeutic neuralgia, or to peripheral nerve injury.

11. The use according to claim 4, wherein the disease, disorder or condition is associated with withdrawal symptoms caused by termination of use of addictive substances, including nicotine containing products such as tobacco, opioids such as heroin, cocaine and morphine, benzodiazepines and benzodiazepine-like drugs and alcohol.

## Patentansprüche

1. Diazabicyclisches Arylderivat, das durch Formel IV wiedergegeben ist 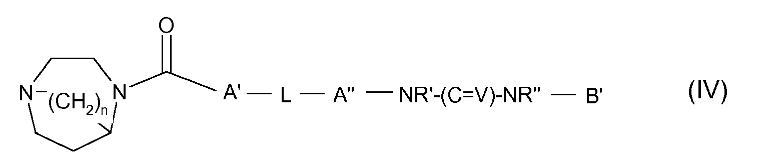 beliebige von seinen Enantiomeren oder eine beliebige Mischung von seinen Enantiomeren oder ein pharmazeutisch verträgliches Additionssalz davon, worin
n 2 ist;
L eine (kovalente) Einfachbindung bedeutet;
A' eine Furanylgruppe bedeutet;
A" eine Phenylgruppe bedeutet;
R' Wasserstoff, C₁₋₆-Alkyl oder -(C=O)-NH-B'- bedeutet;
R" Wasserstoff, C₁₋₆-Alkyl, Phenyl oder Benzyl bedeutet;
V O, S oder NH bedeutet; und
B' C₁₋₆-Alkyl, Phenyl, Benzyl oder Pyridyl bedeutet, wobei diese Phenyl-, Benzyl- und Pyridylgruppen gegebenenfalls ein oder zweifach mit Substituenten substituiert sind, die ausgewählt sind aus der Gruppe bestehend aus Hydroxy, C₁₋₆-Alkoxy, Halogen, Trifluormethyl, Nitro, Amino, C₁₋₆-Alkyl-carbonyl-amino, N-C₁₋₆-Alkyl-amino-carbonyl-amino (N-C₁₋₆-Alkyl-ureido), N,N-Di-C₁₋₆-alkyl-amino-carbonyl-amino (N,N-Di-C₁₋₆-alkyl-ureido) und Sulfamoyl.

2. Diazabicyclisches Arylderivat nach Anspruch 1, welches
1-{4-[5-(1,4-Diaza-bicyclo[3.2.2]nonan-4-carbonyl)-furan-2-yl]-phenyl}-3-ethyl-harnstoff;
1-{4-[5-(1,4-Diaza-bicyclo[3.2.2]nonan-4-carbonyl)-furan-2-yl]-phenyl}-3-phenyl-harnstoff;
1-{4-[5-(1,4-Diaza-bicyclo[3.2.2]nonan-4-carbonyl)-furan-2-yl]-phenyl}-3-(2-nitrophenyl)-harnstoff;
1-{4-[5-(1,4-Diaza-bicyclo[3.2.2]nonan-4-carbonyl)-furan-2-yl]-phenyl}-3-(2-acetylamino-phenyl)-harnstoff;
1-{4-[5-(1,4-Diaza-bicyclo[3.2.2]nonan-4-carbonyl)-furan-2-yl]-phenyl}-3-(2-aminophenyl)-harnstoff;
1-{4-[5-(1,4-Diaza-bicyclo[3.2.2]nonan-4-carbonyl)-furan-2-yl]-phenyl}-3-(5-chlor-2-methoxyphenyl)-thioharnstoff;
1-{4-[5-(1,4-Diaza-bicyclo[3.2.2]nonan-4-carbonyl)-furan-2-yl]-phenyl}-3-(5-chlor-2-methoxyphenyl)-harnstoff;
1-{4-[5-(1,4-Diaza-bicyclo[3.2.2]nonan-4-carbonyl)-furan-2-yl]-phenyl}-3-benzyl-harnstoff;
1-{4-[5-(1,4-Diaza-bicyclo[3.2.2]nonan-4-carbonyl)-furan-2-yl]-phenyl}-1'-benzylamino-carbonyl-3-benzyl-harnstoff;
1-{4-[5-(1,4-Diaza-bicyclo[3.2.2]nonan-4-carbonyl)-furan-2-yl]-phenyl}-1'-benzylamino-carbonyl-3-benzyl-harnstoff;
1-{4-[5-(1,4-Diaza-bicyclo[3.2.2]nonan-4-carbonyl)-furan-2-yl]-phenyl}-3-(2-chlorphenyl)-harnstoff;
1-{3-[5-(1,4-Diaza-bicyclo[3.2.2]nonan-4-carbonyl)-furan-2-yl]-phenyl}-3-phenyl-harnstoff;
1-{4-[5-(1,4-Diaza-bicyclo[3.2.2]nonan-4-carbonyl)-furan-2-yl]-phenyl}-3-(2-fluorphenyl)-harnstoff;
1-{4-[5-(1,4-Diaza-bicyclo[3.2.2]nonan-4-carbonyl)-furan-2-yl]-phenyl}-3-(3-fluorphenyl)-harnstoff;
1-{4-[5-(1,4-Diaza-bicyclo[3.2.2]nonan-4-carbonyl)-furan-2-yl]-phenyl}-3-(2-trifluormethylphenyl)-harnstoff;
1-[2-(3-{4-[5-(1,4-Diaza-bicyclo[3.2.2]nonan-4-carbonyl)-furan-2-yl]-phenyl}-ureido)-phenyl]-3-ethyl-harnstoff;
1-{4-[5-(1,4-Diaza-bicyclo[3.2.2]nonan-4-carbonyl)-furan-2-yl]-phenyl}-3-(3-trifluormethylphenyl)-harnstoff; oder
1-{3-[5-(1,4-Diaza-bicyclo[3.2.2]nonan-4-carbonyl)-furan-2-yl]-phenyl}-3-ethyl-harnstoff ist;
oder ein Enantiomer oder eine Mischung von seinen Enantiomeren oder ein pharmazeutisch verträgliches Additionssalz davon.

3. Pharmazeutische Zusammensetzung umfassend eine therapeutisch wirksame Menge von einem diazabicyclischen Arylderivat nach einem der Ansprüche 1-2, oder einem Enantiomer oder einer Mischung von seinen Enantiomeren, oder einem pharmazeutisch verträglichen Additionssalz davon, zusammen mit wenigstens einem pharmazeutisch verträglichen Träger oder Verdünnungsmittel.

4. Verwendung von einem diazabicyclischen Arylderivat nach einem der Ansprüche 1-2, oder einem Enantiomer oder einer Mischung von seinen Enantiomeren, oder einem pharmazeutisch verträglichen Additionssalz davon, für die Herstellung einer pharmazeutischen Zusammensetzung/eines Medikaments für die Behandlung, Verhütung oder Linderung einer Krankheit oder einer Störung oder eines Zustands eines Säugers, einschließlich eines Menschen, wobei die Krankheit, die Störung oder der Zustand auf die Modulation von cholinergen Rezeptoren anspricht.

5. Verwendung nach Anspruch 4, wobei es sich bei der Krankheit, der Störung oder dem Zustand um Angst, kognitive Störungen, ein Lerndefizit, Gedächtnisdefizite und eine Gedächtnisdysfunktion, die Alzheimer-Krankheit, ein Aufmerksamkeitsdefizit, eine Aufmerksamkeitsdefizit-Hyperaktivitätsstörung, die Parkinson-Krankheit, Chorea Huntington, amyotrophe Lateralsklerose, das Gilles de la Tourette-Syndrom, eine Depression, Manie, manische Depression, Schizophrenie, Zwangsstörungen (OCD), Panikstörungen, Essstörungen wie Anorexia nervosa, Bulimie und Fettleibigkeit, Narkolepsie, Nozizeption, AIDS-Demenz, senile Demenz, periphere Neuropathie, Autismus, Dyslexie, tardive Dyskinesie, Hyperkinesie, Epilepsie, Bulimie, das post-traumatische Syndrom, eine Sozialphobie, Schlafstörungen, Pseudodemenz, das Ganser-Syndrom, das prämenstruelle Syndrom, das Syndrom der späten Lutealphase, das chronische Ermüdungssyndrom, Mutismus, Trichotillomanie und Jetlag handelt.

6. Verwendung nach Anspruch 4, wobei die Krankheit, die Störung oder der Zustand mit Kontraktionen eines glatten Muskels verbunden ist, einschließlich konvulsiver Störungen, Angina pectoris, Frühgeburt, Krämpfe, Diarrhoe, Asthma, Epilepsie, tardiver Dyskinesie, Hyperkinesie, vorzeitiger Ejakulation und Erektionsschwierigkeiten.

7. Verwendung nach Anspruch 4, wobei die Krankheit, die Störung oder der Zustand mit dem endokrinen System zusammenhängt, wie Thyreotoxikose, Phäochromozytom, Hochdruck und Arrhythmien.

8. Verwendung nach Anspruch 4, wobei es sich bei der Krankheit, der Störung oder dem Zustand um eine neurodegenerative Störung handelt, einschließlich vorübergehender Anoxie und induzierter Neurodegeneration.

9. Verwendung nach Anspruch 4, wobei es sich bei der Krankheit, der Störung oder dem Zustand um eine entzündliche Störung handelt, einschließlich entzündlicher Hautstörungen wie Akne und Rosacea, Morbus Crohn, entzündlicher Darmerkrankung, Colitis ulcerosa und Diarrhoe.

10. Verwendung nach Anspruch 4, wobei es sich bei der Krankheit, der Störung oder dem Zustand um milde, mäßige oder auch starke Schmerzen von akutem, chronischem oder wiederkehrendem Charakter, sowie neuropathische Schmerzen und durch Migräne verursachte Schmerzen, post-operative Schmerzen, Phantomschmerzen, neuropathische Schmerzen, chronische Kopfschmerzen, zentrale Schmerzen, Schmerzen im Zusammenhang mit diabetischer Neuropathie, mit post-therapeutischer Neuralgie oder mit peripherer Nervenverletzung handelt.

11. Verwendung nach Anspruch 4, wobei die Krankheit, die Störung oder der Zustand mit Entzugssymptomen verbunden ist, die durch die Beendigung des Gebrauchs von süchtig machenden Substanzen, einschließlich nikotinhaltiger Produkte wie Tabak, Opioide wie Heroin, Kokain und Morphin, Benzodiazepine und Benzodiazepin-artiger Drogen bzw. Arzneimittel und Alkohol, verursacht sind.

## Revendications

1. Dérivé d'aryle diazabicyclique représenté par la Formule IV 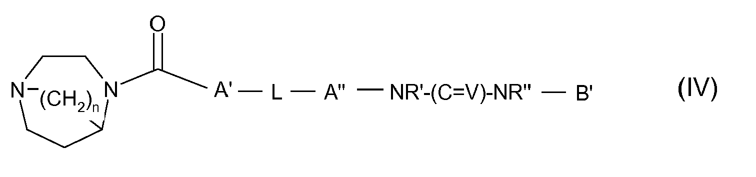 l'un quelconque de ses énantiomères ou un mélange quelconque de ses énantiomères, ou sel d'addition pharmaceutiquement acceptable de celui-ci, dans lequel
n vaut 2 ;
L représente une liaison simple (covalente) ;
A' représente un groupe furanyle ;
A" représente un groupe phényle ;
R' représente un hydrogène, un alkyle en C₁₋₆ ou -(C=O)-NH-B'- ;
R" représente un hydrogène, un alkyle en C₁₋₆, un phényle ou un benzyle ;
V représente O, S ou NH ; et
B' représente un alkyle en C₁₋₆, un phényle, un benzyle ou un pyridyle ;
lesquels groupes phényle, benzyle ou pyridyle sont facultativement substitués une ou deux fois par des substituants choisis dans le groupe constitué par hydroxy, alcoxy en C₁₋₆, halogéno, trifluorométhyle, nitro, amino, alkyl en C₁₋₆-carbonylamino, N-alkyl en C₁₋₆-aminocarbonylamino (N-alkyl en C₁₋₆-uréido), *N,N-*dialkyl en C₁₋₆-aminocarbonylamino (*N,N*-dialkyl en C₁₋₆-uréido) et sulfamoyle.

2. Dérivé d'aryle diazabicyclique selon la revendication 1, qui est
la 1-{4-[5-(1,4-Diaza-bicyclo[3.2.2]nonane-4-carbonyl)-furan-2-yl]-phényl}-3-éthyl-urée ;
la 1-{4-[5-(1,4-Diaza-bicyclo[3.2.2]nonane-4-carbonyl)-furan-2-yl]-phényl}-3-phényl-urée ;
la 1-{4-[5-(1,4-Diaza-bicyclo[3.2.2]nonane-4-carbonyl)-furan-2-yl]-phényl}-3-(2-nitrophényl)-urée ;
la 1-{4-[5-(1,4-Diaza-bicyclo[3.2.2]nonane-4-carbonyl)-furan-2-yl]-phényl}-3-(2-acétylaminophényl)-urée ;
la 1-{4-[5-(1,4-Diaza-bicyclo[3.2.2]nonane-4-carbonyl)-furan-2-yl]-phényl}-3-(2-aminophényl)-urée ;
la 1-{4-[5-(1,4-Diaza-bicyclo[3.2.2]nonane-4-carbonyl)-furan-2-yl]-phényl}-3-(5-chloro-2-méthoxy-phényl)-thiourée ;
la 1-{4-[5-(1,4-Diaza-bicyclo[3.2.2]nonane-4-carbonyl)-furan-2-yl]-phényl}-3-(5-chloro-2-méthoxy-phényl)-urée ;
la 1-{4-[5-(1,4-Diaza-bicyclo[3.2.2]nonane-4-carbonyl)-furan-2-yl]-phényl}-3-benzyl-urée ;
la 1-{4-[5-(1,4-Diaza-bicyclo[3.2.2]nonane-4-carbonyl)-furan-2-yl]-phényl}-1'-benzylaminocarbonyl-3-benzyl-urée ;
la 1-{4-[5-(1,4-Diaza-bicyclo[3.2.2]nonane-4-carbonyl)-furan-2-yl]-phényl}-1'-benzylaminocarbonyl-3-benzyl-urée ;
la 1-{4-[5-(1,4-Diaza-bicyclo[3.2.2]nonane-4-carbonyl)-furan-2-yl]-phényl}-3-(2-chlorophényl)-urée ;
la 1-{3-[5-(1,4-Diaza-bicyclo[3.2.2]nonane-4-carbonyl)-furan-2-yl]-phényl}-3-phényl-urée ;
la 1-{4-[5-(1,4-Diaza-bicyclo[3.2.2]nonane-4-carbonyl)-furan-2-yl]-phényl}-3-(2-fluorophényl)-urée ;
la 1-{4-[5-(1,4-Diaza-bicyclo[3.2.2]nonane-4-carbonyl)-furan-2-yl]-phényl}-3-(3-fluorophényl)-urée ;
la 1-{4-[5-(1,4-Diaza-bicyclo[3.2.2]nonane-4-carbonyl)-furan-2-yl]-phényl}-3-(2-trifluorométhyl-phényl)-urée ;
la 1-[2-(3-{4-[5-(1,4-Diaza-bicyclo[3.2.2]-nonane-4-carbonyl)-furan-2-yl]-phényl}-uréido)-phényl]-3-éthyl-urée ;
la 1-{4-[5-(1,4-Diaza-bicyclo[3.2.2]nonane-4-carbonyl)-furan-2-yl]-phényl}-3-(3-trifluorométhyl-phényl)-urée ; ou
la 1-{3-[5-(1,4-Diaza-bicyclo[3.2.2]nonane-4-carbonyl)-furan-2-yl]-phényl}-3-éthyl-urée ;
ou un énantiomère ou un mélange de ses énantiomères, ou un sel d'addition pharmaceutiquement acceptable de celui-ci.

3. Composition pharmaceutique comprenant une quantité thérapeutiquement efficace d'un dérivé d'aryle diazabicyclique selon l'une des revendications 1-2, ou d'un énantiomère ou d'un mélange de ses énantiomères, ou d'un sel d'addition pharmaceutiquement acceptable de celui-ci, conjointement avec au moins un véhicule ou diluant pharmaceutiquement acceptable.

4. Utilisation d'un dérivé d'aryle diazabicyclique selon l'une des revendications 1-2, ou d'un énantiomère ou d'un mélange de ses énantiomères, ou d'un sel d'addition pharmaceutiquement acceptable de celui-ci, pour la fabrication d'une composition pharmaceutique/médicament pour le traitement, la prévention ou l'atténuation d'une maladie, d'un trouble ou d'une affection chez un mammifère, y compris l'être humain, laquelle maladie, lequel trouble ou laquelle affection réagit à une modulation des récepteurs cholinergiques.

5. Utilisation selon la revendication 4, dans laquelle la maladie, le trouble ou l'affection est une anxiété, des troubles cognitifs, une déficience d'apprentissage, des déficiences et un dysfonctionnement de la mémoire, une maladie d'Alzheimer, une déficience de l'attention, un trouble d'hyperactivité avec déficience de l'attention, une maladie de Parkinson, une maladie de Huntington, une sclérose latérale amyotrophique, un syndrome de Gilles de la Tourette, une dépression, une manie, une manie-dépression, une schizophrénie, des troubles obsessivo-compulsifs (TOC), des troubles paniques, des troubles de l'alimentation tels qu'une anorexie mentale, une boulimie et une obésité, une narcolepsie, une nociception, une démence liée au SIDA, une démence sénile, une neuropathie périphérique, un autisme, une dyslexie, une dyskinésie tardive, une hyperkinésie, une épilepsie, une boulimie, un syndrome post-traumatique, une phobie sociale, des troubles du sommeil, une pseudodémence, un syndrome de Ganser, un syndrome prémenstruel, un syndrome de phase lutéale tardive, un syndrome de fatigue chronique, un mutisme, une trichotillomanie et un décalage horaire.

6. Utilisation selon la revendication 4, dans laquelle la maladie, le trouble ou l'affection est lié(e) à des contractions des muscles lisses, incluant des troubles convulsifs, une angine de poitrine, un travail prématuré, des convulsions, une diarrhée, un asthme, une épilepsie, une dyskinésie tardive, une hyperkinésie, une éjaculation prématurée et une difficulté d'érection.

7. Utilisation selon la revendication 4, dans laquelle la maladie, le trouble ou l'affection est lié(e) au système endocrinien, comme une thyrotoxicose, un phéochromocytome, une hypertension et des arythmies.

8. Utilisation selon la revendication 4, dans laquelle la maladie, le trouble ou l'affection est une maladie neurodégénérative, incluant une anoxie transitoire et une neurodégénérescence induite.

9. Utilisation selon la revendication 4, dans laquelle la maladie, le trouble ou l'affection est un trouble inflammatoire, incluant des troubles cutanés inflammatoires tels qu'une acné et une rosacée, une maladie de Crohn, une affection intestinale inflammatoire, une colite ulcérative et une diarrhée.

10. Utilisation selon la revendication 4, dans laquelle la maladie, le trouble ou l'affection est une douleur légère, modérée ou même sévère, de caractère aigu, chronique ou récurrent, ainsi qu'une douleur neuropathique et une douleur provoquée par une migraine, une douleur postopératoire, une douleur de membre fantôme, une douleur neuropathique, une céphalée chronique, une douleur centrale, une douleur liée à une neuropathie diabétique, à une névralgie post-thérapeutique ou à une lésion du nerf périphérique.

11. Utilisation selon la revendication 4, dans laquelle la maladie, le trouble ou l'affection est associé(e) à des symptômes de sevrage causés par l'arrêt de l'utilisation de substances toxicomanogènes, incluant les produits contenant de la nicotine tels que le tabac, les opioïdes tels que l'héroïne, la cocaïne et la morphine, les benzodiazépines et substances médicamenteuses de type benzodiazépine, et l'alcool.
